# EUROPEAN PATENT APPLICATION

(11) **EP 4 374 847 A1**
(43) Date of publication of application: **29.05.2024**
(21) Application number: 22845785.9
(22) Date of filing: 07.07.2022
(51) Int. Cl.: A61K 8/9789, A61K 8/9794, A61Q 19/00, A61Q 19/08, C12Q 1/686, C12Q 1/6876

(54) **OBP PROMOTER**

(30) Priority: 21.07.2021 JP 2021120800; 30.06.2022 JP 2022106314
(71) Applicant: Shiseido Company, Ltd., Chuo-ku Tokyo 104-0061 (JP)
(72) Inventor: NAKANISHI, Shinobu, Tokyo 104-0061 (JP); IIJIMA, Ayami, Tokyo 104-0061 (JP)
(74) Representative: Cabinet Beau de Loménie
(86) International application number: PCT/JP2022/026895
(87) International publication number: WO 2023/002851

(57) **Abstract**

The present disclosure relates to an agent that promotes expression of OBP, and to a method of using it. More specifically, it provides an OBP expression promoter comprising a plant extract as an active ingredient. The OBP expression promoter may be used for promoting expression of OBP in skin with reduced OBP activity (epidermal cells and/or keratinocytes), or for reducing damage to skin by harmful substances to enhance the moisturizing function and/or barrier function of the skin.

## Description

### FIELD

The present invention relates to an expression promoter for OBP (Odorant Binding Protein) and to a method for screening for substances that promote or suppress expression of OBP.

### BACKGROUND

To date, a large number of substances have been identified as being harmful to animals such as humans. Some substances, however, have not yet been identified as being either harmful or not, and have not been definitively characterized in regard to damaging effects on the body. Examples of such substances may include small molecules that are invisible to the naked eye, volatilized substances, and vaporous substances. Persons are often unaware when coming into contact with such substances, and continued chronic exposure without realizing it can potentially result in gradual damage to the body.

Animal bodies have methods of protection from such harmful substances, by mechanisms of discharging through body fluids such as tears and nasal mucus. For example, the nose is not only a sensor for detecting the odors of harmful substances, but also has a protective function of capturing harmful substances by nasal mucus which covers the mucous membrane in the nasal cavity. Nasal mucus contains a protein substance known as OBP (odorant binding protein), and it has been suggested that scavenging of harmful substances by OBP may help prevent outside harmful substances from entering the body. OBP has also been reported to be present in tears, the prostate and the mammary glands (NPLs 1 to 3).

With the skin, the defense mechanism is one in which larger molecules in the stratum corneum prevent entrance into the skin interior by providing a barrier function. However, it is possible for smaller molecules scavenged by OBP to pass through keratinocytes. The details regarding how such molecules are protected from by the skin still remain to be uncovered.

### [CITATION LIST]

### [PATENT LITERATURE]

[PTL 1] Japanese Unexamined Patent Publication HEI No. 11-286423
[PTL 2] Japanese Unexamined Patent Publication HEI No. 11-286428
[PTL 3] Japanese Unexamined Patent Publication No. 2012-32287

### [NON PATENT LITERATURE]

[NPL 1] FEBS Journal 273(2006): 5131-5142
[NPL 2] Free Radical Research(2014)48(7): 814-822
[NPL 3] Biochemical Journal (2001) 356(1): 129-135

### SUMMARY

### [TECHNICAL PROBLEM]

It is one object of the invention to provide a substance that promotes expression of OBP (odorant binding protein). It is another object of the invention to provide a screening method for substances that promote or suppress expression of OBP.

### [SOLUTION TO PROBLEM]

The present inventors have found, surprisingly, that the substances cabbage rose extract, cherry blossom extract and lily extract promote expression of OBP. Having conducted much research based on this finding, the present inventors have created the present invention relating to an OBP expression promoter.

The present application encompasses the following inventions:
(1) An OBP (odorant binding protein) expression promoter comprising a substance selected from the group consisting of lily extract, cabbage rose extract and cherry blossom extract as an active ingredient.
(2) The OBP expression promoter according to (1) above, which is to be used for promoting expression of OBP in epidermal cells and/or keratinocytes.
(3) The OBP expression promoter according to (1) or (2) above, which is to be used for promoting expression of OBP in skin with reduced OBP activity.
(4) The OBP expression promoter according to any one of (1) to (3) above, which is to be used for enhancing the moisturizing function and/or barrier function of skin.
(5) The OBP expression promoter according to any one of (1) to (4) above, which is to be used for reducing damage to skin by harmful substances.
(6) The OBP expression promoter according to (5) above, wherein the harmful substance is selected from the group consisting of aldehydes, fatty acids and air pollutants.
(7) A cosmetic method or non-therapeutic method that includes application of an OBP expression promoter comprising a substance selected from the group consisting of lily extract, cabbage rose extract and cherry blossom extract as an active ingredient.
(8) Use of a substance selected from the group consisting of lily extract, cabbage rose extract and cherry blossom extract for promoting expression of OBP.
(9) An external preparation for skin comprising an OBP expression promoter comprising a substance selected from the group consisting of lily extract, cabbage rose extract and cherry blossom extract as an active ingredient.
(10) An inhibitor of harmful substances to skin, comprising a substance selected from the group consisting of lily extract, cabbage rose extract and cherry blossom extract as an active ingredient.
(11) A method for screening substances that promote or suppress expression of OBP, the method comprises a step of contacting cells with a substance, a step of measuring the expression level of OBP in the cells, and a step of determining the change in OBP expression level due to the presence or absence of the substance.

### [ADVANTAGEOUS EFFECTS OF INVENTION]

The present invention provides an OBP expression promoter. The OBP expression promoter may be used for promoting expression of OBP in skin with reduced OBP activity (epidermal cells and/or keratinocytes), or for reducing damage to skin by harmful substances to enhance the moisturizing function and/or barrier function of the skin. The screening method of the invention allows identification of substances that are useful as OBP expression promoters.

### BRIEF DESCRIPTION OF DRAWINGS

Fig. 1 is a pair of graphs showing observed transcription of OBP (OBP2A and OBP2B) in keratinocytes and a 3D skin model.
Fig. 2 is a graph showing the effect of applying t-2-nonenal on viability of OBP2A/OBP2B-knockdown keratinocytes.
Fig. 3 is a graph showing the effect of applying oleic acid on viability of OBP2A/OBP2B-knockdown keratinocytes.
Fig. 4 is a graph showing the effect of applying palmitoleic acid on viability of OBP2A/OBP2B-knockdown keratinocytes.
Fig. 5 is a diagram showing simulated results for scavenging of t-2-nonenal by OBP.
Fig. 6 is a diagram showing simulated results for scavenging of oleic acid by OBP.
Fig. 7 is a diagram showing simulated results for scavenging of palmitoleic acid by OBP.
Fig. 8 is a graph showing increase in OBP transcription induced by plant extracts.
Fig. 9 is a graph showing increase in filaggrin transcription induced by cherry blossom extract.
Fig. 10 is a graph showing increase in kallikrein 10 transcription induced by cherry blossom extract.
Fig. 11 is a graph showing increase in caspase 14 transcription induced by cherry blossom extract.
Fig. 12 is a graph showing increase in filaggrin transcription induced by lily extract.
Fig. 13 is a graph showing increase in kallikrein 10 transcription induced by lily extract.
Fig. 14 is a graph showing increase in caspase 14 transcription induced by lily extract.

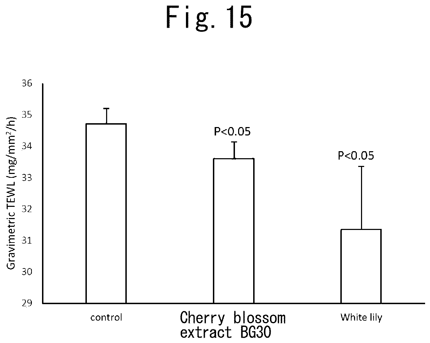

Fig. 15 is a graph showing improvement in TEWL by cherry blossom extract and lily extract.

### DESCRIPTION OF EMBODIMENTS

### [Odorant Binding Protein]

OBP (Odorant Binding Protein) is known to be present in numerous animals and insects and to have a structure depending on the specific type, but it is generally a 10 to 30 kDa protein having an α-helix structure with 6 conserved cysteines bonded by three disulfide bonds. In vertebrates, OBP is part of the lipocalin family, having a β-barrel motif formed by antiparallel β-sheets. Humans have three types of OBP: OBP2A, OBP2B and LCN1. OBP2A is present in the nasal mucus and has high affinity for fatty acids and aldehydes. OBP2B is present in the prostate and mammary glands, while LCN1 is present in tears.

The function of OBP has not been fully established, but based on reports that it functions as a scavenger for 4-hydroxy-2-nonenal in mammalian nasal mucosa (NPL 1), that bovine OBP monomeric overexpression protects *E. coli* from chemical substance-induced oxidative stress (NPL 2), and that human tear lipocalin functions in cell cultures as an oxidative stress-induced scavenger of potentially harmful lipid peroxidation products (NPL 3), it has been suggested that it may play a protective role against external harmful substances.

During the course of ongoing research, the present inventors have found that OBP is also present in skin. Infiltration of harmful substances of high molecular weight into the skin is prevented by the stratum corneum, but smaller molecular weights can potentially infiltrate into the skin through the stratum corneum. OBP present in the skin is thought to play a protective role against such small molecules.

### [OBP (Odorant binding protein) expression promoter]

One aspect of the invention relates to an OBP (odorant binding protein) expression promoter. According to several embodiments, the OBP expression promoter may comprise a substance selected from the group consisting of cabbage rose extract, cherry blossom extract and lily extract as an active ingredient. Promoting expression of OBP means increasing the amount of transcription product of OBP and/or increasing the amount of protein.

A substance that can be used as an active ingredient in an OBP expression promoter may be a plant extract selected from the group consisting of cabbage rose extract, cherry blossom extract and lily extract. According to several embodiments, a plurality of different plant extracts may be used in combination.

The "plant extract" to be used for the invention may be from any part such as the entire plant, leaves, flowers, stems, roots or grains. When flowers are used, the flowering period and size are not particularly restricted, and they may include the petals or calyx, or the entirety. These plant extracts may be extracted from plant materials by methods known to those skilled in the art. A plant extract can be obtained using any method publicly known to those skilled in the art, such as an extraction method using solvents or a method that includes pulverizing and pressing steps.

The plant extraction site is first washed to remove contaminants if necessary and used either directly or after drying, with chopping or pulverizing as necessary, and then contacted with an extraction solvent for extraction. The extraction may be carried out by contact with an extraction solvent by an common method such as immersion, or supercritical extraction or steam distillation may be employed instead.

Extraction solvents include water; lower alcohols such as methanol, ethanol and propanol and higher alcohols such as oleyl alcohol, stearyl alcohol and octyldodecanol; polyhydric alcohols such as ethylene glycol, 1,3-propanediol, 1,3-butylene glycol and glycerin; esters such as ethyl acetate, butyl acetate, methyl propionate and glyceryl trioctanoate; ketones such as acetone and methyl ethyl ketone; ethers such as ethyl ether, isopropyl and ether; and hydrocarbon-based solvents such as *n*-hexane, toluene and chloroform, any of which may be used alone or in mixtures of two or more.

The mixing ratio when using a mixed solvent is preferably in the range of 1:1 to 25:1 (volume ratio, same hereunder) for a mixed solvent of water and ethyl alcohol, in the range of 1:1 to 15:1 for a mixed solvent of water and glycerin, or in the range of 1:1 to 15:1 for a mixed solvent of water and 1,3-propanediol or 1,3-butylene glycol.

The pH used when preparing the extract is not particularly restricted but is generally preferred to be in the range of pH 3 to 9. If necessary, an alkaline adjustor such as sodium hydroxide, sodium carbonate or potassium hydroxide or an acidic adjustor such as citric acid, hydrochloric acid, phosphoric acid or sulfuric acid may be added to the extraction solvent for adjustment to the desired pH.

The extraction conditions such as the extraction temperature and extraction time are not limited as they will differ depending on the type of solvent used and the pH, but when the solvent is water, 1,3-butylene glycol or a mixture of water and 1,3-butylene glycol, for example, the extraction temperature may be in the range of 0°C to 90°C and the extraction time may be 0.5 hour to 7 days.

The extraction site may also be subjected to hydrolysis if necessary, before the extraction treatment or simultaneously with the extraction treatment. This can improve skin irritation, effectiveness or storage stability of the extract to allow the extract to be more effectively utilized.

A commercially available plant extract may also be used.

According to several embodiments, the OBP expression promoter of the present disclosure may include a cosmetic, quasi drug, drug or functional food in the composition. For addition to a cosmetic, the addition may be to any cosmetic that is to be applied to skin, such as sunscreen, cosmetic water, essence, emulsion, beauty cream or aftercare lotion. The composition may be administered to a subject with reduced OBP activity, for example, to prevent or ameliorate damage caused by harmful substances to the skin.

The OBP expression promoter of the invention may have optional compounding ingredients added as necessary and appropriate, such as are used in cosmetics and pharmaceuticals, within ranges that do not interfere with its effect. Examples of arbitrary compounding ingredients include oils, surfactants, powders, coloring materials, water, alcohols, thickeners, chelating agents, silicones, antioxidants, ultraviolet absorbers, humectants, perfumes, drug components, antiseptic agents, pH adjustors and neutralizing agents. Anti-inflammatory components and whitening components may also be included, as examples of other components.

The content of an extract to be included in the OBP expression promoter may be set as appropriate by a person skilled in the art.

According to several embodiments, the OBP expression promoter may be used for promoting expression of OBP in skin tissue, and especially epidermal cells and/or keratinocytes.

The OBP expression promoter of the invention may also be used in a cosmetic method to promote expression of OBP in epidermal cells and/or keratinocytes by application onto skin. The method of usage and dosage for an external preparation for skin according to this disclosure to be used in a cosmetic method is not particularly restricted and may be appropriately established depending on the dosage form or the condition of the skin to be treated, but typically a suitable dose, e.g. from 0.1 ml to 1 ml per cm² is rubbed directly onto the skin or absorbed in gauze and applied onto the skin, several (1-5) times per day.

According to several embodiments, the OBP expression promoter may be used for promoting expression of OBP in skin with reduced OBP activity. Causes of reduced OBP activity include aging and fatigue, for example.

When OBP activity has been reduced by aging or fatigue, for example, this increases the possibility that harmful substances will fail to be adequately scavenged by OBP and may infiltrate into the skin. The present invention is also useful from the viewpoint of preventing damage to skin with reduced OBP activity by harmful substances to the skin. Reduction of OBP activity, as referred to herein, means that the amount, expression level and/or function of OBP is reduced, and for example, that the OBP gene expression level and/or OBP protein level is reduced with a statistically significant difference (Dunnett's test, for example) at a significance level of 5%, or reduced by 5% or more, 10% or more, 20% or more, 30% or more, 40% or more, 50% or more, 60% or more, 70% or more, 80% or more, 90% or more, or 100%, for example, compared to a state without reduced activity. A person skilled in the art may evaluate reduction in OBP activity using a publicly known method.

According to several embodiments, the OBP expression promoter may be used for enhancing the moisturizing function and/or barrier function of skin.

Of the four constituent layers of the epidermis, the stratum corneum situated as the outermost layer on the outermost side of the skin has a barrier function that prevents infiltration of foreign matter from the outside and a moisturizing function that inhibits transpiration of moisture from the body. Reduced barrier function or moisturizing function of the stratum corneum can lead to various problems such as skin roughening and dry skin. When skin barrier function is lost, skin moisture is also lost and infiltration of exterior foreign matter occurs, creating a condition of sensitive and dry skin and skin roughening, and potentially resulting in dermatitis and allergy. Indicators for skin barrier function include, for example, transepidermal water loss (TEWL), and the degree of penetration into skin using an indicator substance such as a pigment. TEWL of skin can be measured using a device such as a VapoMeter.

By promoting expression of OBP it is possible to improve the moisturizing function and/or barrier function of skin, by preventing damage to skin caused by harmful substances to the skin.

According to several embodiments, the OBP expression promoter may be used for reducing damage to skin by harmful substances.

Skin damage indicates a deteriorated skin condition caused by harmful substances, examples including thinning of the skin of animals such as humans, visibility of pores, bowl-shaped enlargement of pores, disturbance of texture and/or parakeratosis. However, skin damage is not so limited and will differ depending on the type of harmful substances to the skin.

Harmful substances may include aldehydes, fatty acids and air pollutants, for example. Examples of aldehydes include, but are not limited to, *n*-hexanal, n-heptanal, n-nonanal, *n-*decanal, undecanal, *n*-dodecanal, benzaldehyde, α-methyl-3,4-(methylenedioxy)-hydrocinnamaldehyde and 2-methyl-3-(4-*tert*-butylphenyl)-propanal. Examples of fatty acids include, but are not limited to, octanoic acid (caprylic acid), decanoic acid (capric acid), dodecanoic acid (lauric acid), tetradecanoic acid (myristic acid), hexadecanoic acid (palmitic acid), 12-bromododecanoic acid (BrC12-Ac) and 15-bromopentadecanoic acid (BrC15-Ac). Examples of air pollutants included, but are not limited to, sulfur oxides (SOx), nitrogen oxides (NOx), photochemical oxidants (Ox), particulate matter (PM), suspended particulate matter (SPM) and microparticulate matter (PM2.5). Examples of harmful substances are also listed elsewhere throughout the present specification.

### [Cosmetic method and non-therapeutic method]

One aspect of the invention relates to a cosmetic method or non-therapeutic method that includes application of an OBP expression promoter. According to several embodiments, the OBP expression promoter may comprise a substance selected from the group consisting of cabbage rose extract, cherry blossom extract and lily extract as an active ingredient. The method is for the purpose of beautifying, and not for use in treatment by a doctor or health care professional. The target of application may be a mammal, and especially a human, for example. The site of application may be skin on the face, neck, scalp, arms or legs. The application may be in any form such as coating using the hand or an applicator. The number, frequency and amount of application may be set as appropriate depending on the desired effect.

### [Use]

One aspect of the invention relates to use of a substance selected from the group consisting of cabbage rose extract, cherry blossom extract and lily extract for promoting expression of OBP. From a different viewpoint, one aspect of the invention relates to use of a substance selected from the group consisting of cabbage rose extract, cherry blossom extract and lily extract for use in promoting expression of OBP. The data disclosed herein demonstrate that substances selected from the group consisting of cabbage rose extract, cherry blossom extract and lily extract are useful for promoting expression of OBP. The plant extract may therefore be used to prepare a composition for use in promoting expression of OBP. One aspect of the invention therefore relates to use of a substance selected from the group consisting of cabbage rose extract, cherry blossom extract and lily extract for production of a composition (such as an external preparation for skin) for promoting expression of OBP.

### [External preparation for skin]

One aspect of the invention relates to an external preparation for skin comprising an OBP expression promoter comprising a substance selected from the group consisting of cabbage rose extract, cherry blossom extract and lily extract as an active ingredient.

According to several embodiments, the OBP expression promoter may be compounded as an external preparation for skin that can be directly applied to skin. Such an external preparation for skin may also contain optional compounding ingredients used in cosmetics or pharmaceuticals in addition to the plant extract, as necessary and appropriate. The external preparation for skin may be prepared in any form such as an external solid preparation, external powder, external liquid, liniment, spray agent, topical aerosol, pump spray agent, ointment, cream, gel, medical patch, tape preparation or poultice.

The dosage form of the external preparation for skin of the disclosure is not particularly restricted, and for example, it may be in any desired form such as a solution system, a solubilized system, an emulsified system, a powder-dispersed system, a water-oil two-layer system, a water-oil-powder three-layer system, an ointment, gel, aerosol, or the like. The form of use is also not particularly restricted, and for example, any desired form such as cosmetic water or an emulsion, cream, essence, jelly, gel, ointment, pack, mask, foundation or the like may be employed.

### [Inhibitor of harmful substances to skin]

One aspect of the invention relates to an inhibitor of harmful substances to skin, comprising a substance selected from the group consisting of cabbage rose extract, cherry blossom extract and lily extract as an active ingredient.

According to the invention, a harmful substance to the skin is a substance that causes damage to skin but whose activity is reduced by OBP, such as OBP2A or OBP2B. Reduction of activity by OBP means that when OBP is present, the amount, expression level and/or skin-damaging function of the harmful substance to the skin is reduced with a statistically significant difference (Dunnett's test, for example) at a significance level of 5%, or reduced by 5% or more, 10% or more, 20% or more, 30% or more, 40% or more, 50% or more, 60% or more, 70% or more, 80% or more, 90% or more or 100%, for example, compared to when OBP is not present. For example, if the cell viability is lower when using OBP-knockdown skin cells than when using non-knockdown skin cells, the substance can be detected as a harmful substance to skin.

OBP-knockdown skin cells are skin cells such as keratinocytes that have impaired or reduced OBP expression by knockdown treatment of the genes of either or both OBP2A and OBP2B. Knockdown treatment may be by any publicly known technology such as described in Sci Rep. 2018 Oct 23; 8(1): 15610, for example. Non-knockdown skin cells are skin cells with normal OBP expression, without knockdown treatment of the OBP genes treated for knockdown in the knockdown cells. That OBP expression has been impaired or reduced by knockdown treatment means that the expression level of the OBP gene and/or the OBP protein level in the knockdown-treated cells is reduced with a statistically significant difference (Dunnett's test, for example) at a significance level of 5%, or reduced by 5% or more, 10% or more, 20% or more, 30% or more, 40% or more, 50% or more, 60% or more, 70% or more, 80% or more, 90% or more, or 100%, for example, compared to non-knockdown cells.

Harmful substances include, but are not limited to, the harmful substances mentioned above, as well as substances with high affinity for OBP, including malodorous substances such as formaldehyde, acetaldehyde, propionic acid, normal-valeric acid, normal-butyric acid, isovaleric acid, ethyl acetate, methyl isobutyl ketone, isobutanol, or the aldehydes propionaldehyde, butylaldehyde, isobutylaldehyde, valeraldehyde, isovaleraldehyde and nonenal, stress odor substances such as allylmercaptane and dimethyltrisulfide, and fatty acids, including unsaturated fatty acids such as oleic acid and palmitoleic acid, or the publicly known substances listed as OBP ligands in Biochemistry. 2002 Jun 11; 41(23):7241-52, for example.

The present inventors have identified oleic acid, palmitoleic acid and nonenal as harmful substances to the skin. Oleic acid and palmitoleic acid have been reported to provoke pore enlargement or bowl-shaped enlargement of pores, disturbed texture and parakeratosis (J Invest Dermatol. 2005 May; 124(5): 1008-13, Br J Dermatol. 2009 Jan; 160(1):69-74). Nonenal (2-nonenal), referred to throughout the present specification, is the *trans-form* (t-2-nonenal), which is reported to have a connection with aging odor and skin yellowing (PTLs 1 to 3), and the present inventors have found that nonenal exhibits a skin-damaging effect including skin thinning (see Examples).

### [Screening method]

One aspect of the invention relates to a method for screening substances that promote or suppress expression of OBP. According to several embodiments, the screening method of the invention may comprise a step of contacting cells with a substance, a step of measuring the expression level of OBP in the cells, and a step of determining the change in OBP expression level due to the presence or absence of the substance.

The cells may be epidermal cells or keratinocytes, for example. Contact between the cells and test substance may be accomplished by adding the test substance to culture medium of the cells, for example. The OBP expression level may be measured by any method publicly known to those skilled in the art. For example, OBP mRNA level can be quantified by RT-PCR, or OBP protein level can be quantified by ELISA or Western blotting. When the presence or absence of a substance has caused an observable change in OBP expression level, the test substance is evaluated to promote expression of OBP if the expression level has increased, or the test substance is evaluated to suppress expression of OBP if expression has decreased.

A substance that promotes or suppresses expression of OBP may be any substance such as a plant extract, low molecular compound, high molecular polymer, protein, peptide, antibody, enzyme, nucleic acid, sugar chain or lipid, included in a compound library or an extract library from a microbe or animal or plant, or a composition containing any of these. Examples of plant extract compositions include aloe extract, ginkgo extract, oolong tea extract, turmeric extract, turmeric rhizome extract, usubasaishin rhizome/root extract, edelweiss extract, olive leaf extract, hydrolyzed silk powder, *Artemisia capillaris* extract, *Artemisia capillaris* flower extract, licorice extract, raspberry extract, gardenia extract, mulberry extract, gentian root extract, geranium herb extract, tea extract, coffee extract, sesame extract, rice bran extract, carambola leaf extract, wild ginger extract, cherry flower leaf extract, *Tremella fuciformis* polysaccharide, star fruit leaf extract, yarrow extract, sage extract, sage leaf extract, senkyu extract, corn extract, dokudami extract, tomato extract, parsley extract, grape seed extract, safflower extract, peony extract, magwa root bark extract, madonna lily root extract, evening primrose seed extract, lily extract, rooibos extract and rosemary extract.

All of the publications mentioned throughout the present specification are incorporated herein in their entirety by reference. The Examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### EXAMPLES

The present invention will now be explained in greater detail by examples. However, the invention is in no way limited by the Examples.

### Example 1: Expression of OBP in skin

Keratinocyte human epidermal cells were prepared by culturing in Humedia-KG2 (Kurabo Industries, Ltd.) medium at 37°C. For construction of a three-dimensional epidermal model, human epidermal cells (2.2 × 10⁵/500 µl) diluted in CnTPrime medium (CELLnTEC) were seeded in a cell culture insert coated with Cell Start (Invitrogen Life Technologies) solution diluted 50-fold in DPBS (ϕ12 mm, porous membrane mean pore size: 0.4 µm), and 1 ml of CnTPrime medium (CELLnTEC) solution was added under the insert prior to culturing for 3 days at 37°C. The medium on the insert was then replaced with 500 µl of CnT-PR-3D medium (CELLnTEC) containing vitamin C (50 µg/ml) while the medium under the insert was replaced with 1 ml of the same, and culturing was carried out for 1 day at 37°C. After one day, the medium on the insert was aspirated, and the medium under the insert was replaced with 500 µl of CnT-PR-3D medium (CELLnTEC) with added vitamin C (50 µg/ml), and culturing was continued for 1 day at 37°C. This procedure was carried out for 8 days to construct a three-dimensional epidermal model.

ISOGEN (Nippon Gene Co., Ltd.) was used to extract RNA from epidermal cells prepared as described above and differentiated for 0, 24 or 48 hours under conditions with Ca added at 1.8 mM to Humedia-KG2 (Kurabo Industries, Ltd.) medium, and from a three-dimensional epidermal model differentiated for 7 days or 14 days, and then SuperScript IV VILO MasterMix (Thermo FisherScientific) was used to prepare cDNA, and OBP expression was analyzed by qPCR using SYBRTM Green PCR MasterMix (Thermo FisherScientific).

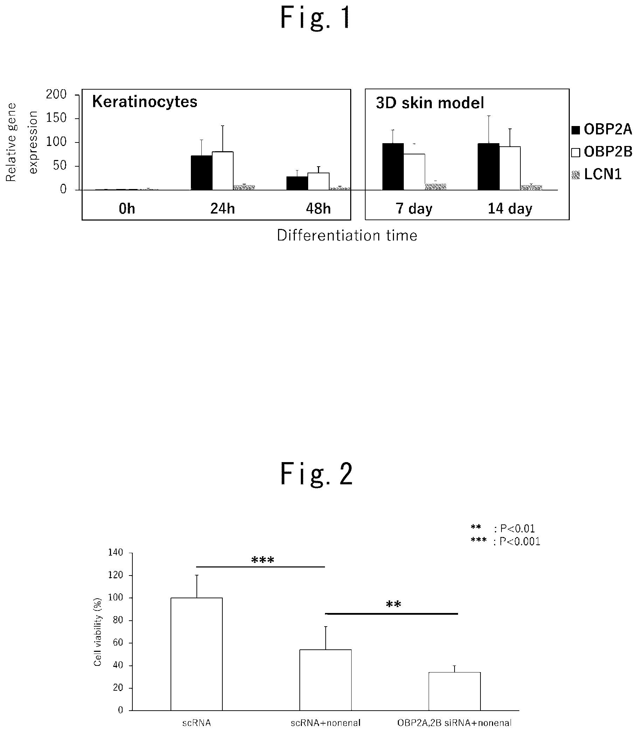

The results are shown in Fig. 1. Fig. 1 shows that transcription of OBP such as OBP2A and OBP2B was confirmed in both the cultured keratinocytes and the three-dimensional epidermal model, indicating the presence of OBP in the skin.

### Example 2: Analysis of effect of OBP knockdown on cell viability

After adding 10 µM of OBP2A and OBP2B siRNA to a mixed solution of Lipofectamine RNAiMAX Transfection Reagent (Thermo FisherScientific) and Opti-MEM I Reduced SerumMedia (Thermo FisherScientific), the mixture was added to epidermal cells cultured by the method described above, and culturing was carried out for 24 hours to prepare OBP2A/OBP2B double-knockdown cells. The control cells were cultured under the same conditions, but without knockdown treatment, and differentiated for 24 hours.

The cultured and differentiated double-knockdown cells and control cells were measured for OBP protein levels by ELISA using a Human odorant binding protein 2A ELISA Kit (MYBioSource) and Human odorant binding protein 2B ELISA Kit (MYBioSource), by which it was confirmed that the protein levels had decreased by 47% for OBP2A and 60% for OBP2B.

Different types of samples including aldehydes such as nonenal and fatty acids such as oleic acid or palmitoleic acid were prepared as examples of harmful substances to the skin.

**[Table 1]**

| Candidate harmful substance to skin | Structure | Preparation method |
|---|---|---|
| Nonenal (*t*-2-nonenal) | 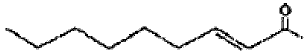 | Dissolution of "*t*-2-Nonenal" by Toronto Research Chemicals Inc. in ethanol to 1 M concentration. |
| Oleic acid | 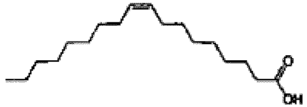 | Dissolution of "Oleic acid" by Nacalai Tesque, Inc. in ethanol to 1 M concentration. |
| Palmitoleic acid | 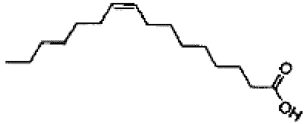 | Dissolution of "Palmitoleic acid" by Toronto Research Chemicals Inc. in ethanol to 1 M concentration. |

The differentiated and cultured control cells and double-knockdown cells were used to evaluate the effects of harmful substances to the skin. The cell viabilities were measured by the method described above at 24 hours following addition in the system with control cells alone, the system with harmful substance added to control cells and the system with harmful substance added to double-knockdown cells. The harmful substance was nonenal at 25 µM concentration, oleic acid at 100 µM concentration or palmitoleic acid at 50 µM concentration.

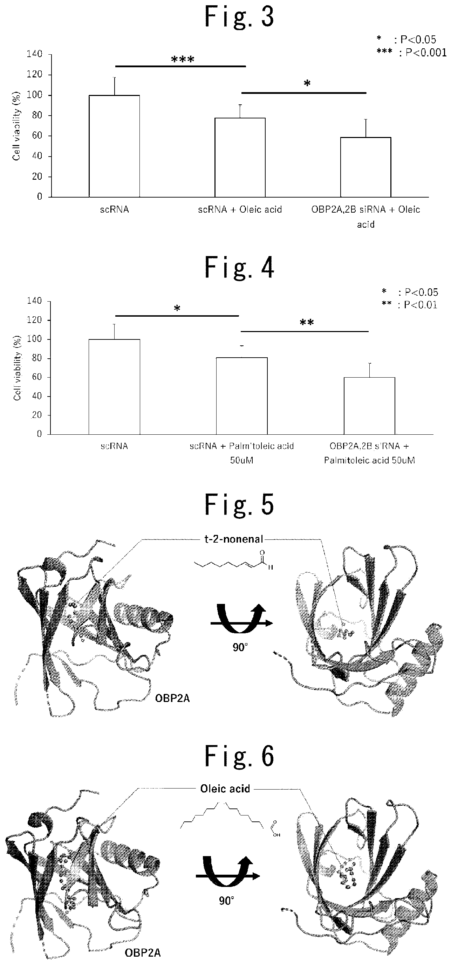

The results are shown in Figs. 2 to 4. Fig. 2 shows that the viability significantly decreased when nonenal was added to the scRNA cells, but that an even greater reduction in cell viability was observed with addition of nonenal to the OBP knockdown cells. These results demonstrated that nonenal acts as a harmful substance to skin, lowering cell viability. Using the same method, Figs. 5 and 6 also show that oleic acid and palmitoleic acid act as harmful substances to the skin, lowering cell viability.

### Example 3: Analysis of scavenging of harmful substance to skin by OBP.

Structural analysis of OBP and the three substances (nonenal, oleic acid and palmitoleic acid) was carried out by simulation using Autodock (4.2.6) and Pymol (2.2.0). The results are shown in Figs. 5 to 7. Figs. 5 to 7 show that all of the substances are scavenged into the binding pocket of OBP.

### Example 4: Evaluation of plant extract-induced promotion of OBP expression

Several plant extracts, specifically cabbage rose extract, cherry blossom extract and lily extract, were examined for their effects of promoting OBP transcription in cells.

The cabbage rose extract used was an extract from cabbage rose flowers (ROSA CENTIFOLIA FLOWER EXTRACT, BUTYLENE GLYCOL, WATER) purchased from Maruzen Pharmaceuticals Co., Ltd. (trade name: Bara Extract BG).

The cherry blossom extract used was an extract with addition of 1,3-butylene glycol to the filtrate obtained by extraction from satozakura (*Prunus lannesiana* Wils. cv. Sekiyama (Rosaceae)), as a product purchased from Oryza Oil & Fat Chemical Co., Ltd. (trade name: CosmeHerbest^{R}) Sakura). The product is obtained by adding ethanol to satozakura (*Prunus lannesiana* Wils. cv. Sekiyama (Rosaceae)) flowers to remove the aromatic components and oils and then adding purified water and filtering, adding 1,3-butylene glycol to the obtained filtrate, and filtering again.

The lily extract used was Chinese White Lily extract (Glycerin, Butylene Glycol, Water, Lilium Candidum Flower Extract) obtained by microwave extraction, purchased from Croda Co. (trade name: Phytofleur^{R} Lily NP).

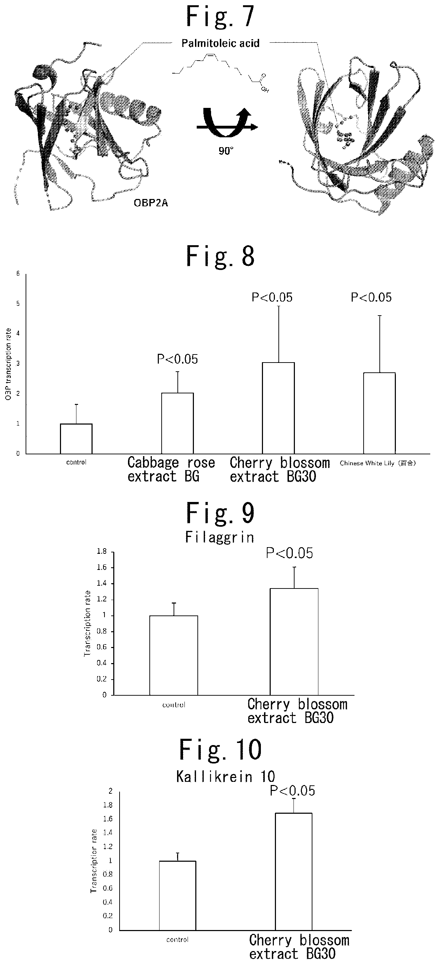

The human epidermal cells were cultured in Humedia-KG2 (Kurabo Industries, Ltd.) medium at 37°C to full confluence, and then each plant extract was added to the medium. Humedia-KG2 medium added in an equal amount as solvent was used as the control. Culturing was carried out at 37°C for 24 hours after addition, and the total RNA was extracted from the cells and used to prepare cDNA by reverse transcription, after which the OBP gene was analyzed by quantitative PCR. The results are shown in Fig. 8. The values are represented as relative values with respect to 1 for the control. Statistical analysis was performed by a two-sided t-test with each plant extract against the control.

The results clearly showed that each plant extract increases OBP gene transcription compared to the control. The plant extracts can therefore be used as OBP expression promoters.

### Example 5: Amelioration of skin barrier function by cherry blossom extract

The effect of cherry blossom extract on known marker genes for skin barrier function was examined. The marker genes selected were filaggrin (see "Filaggrin and Skin Barrier Function" doi: 10.1159/000441539, for example), kallikrein 10 (see "Epidermal Barriers" doi: 10.1101/cshperspect.a018218, for example) and caspase 14 (see "Phospho-ΔNp63α regulates AQP3, ALOX12B, CASP14 and CLDN1 expression through transcription and microRNA modulation" doi: 10.1016/j.febslet. 2013.09.023, for example).

The human epidermal cells were cultured in Humedia-KG2 (Kurabo Industries, Ltd.) medium at 37°C to full confluence, and then cherry blossom extract BG30 was added to the medium. Humedia-KG2 medium added in an equal amount as solvent was used as the control. Culturing was carried out at 37°C for 24 hours after addition, and the total RNA was extracted from the cells and used to prepare cDNA by reverse transcription, after which each gene (filaggrin, kallikrein 10 or caspase 14) was analyzed by quantitative PCR. The results are shown in Figs. 9 to 11. The values are represented as relative values with respect to 1 for the control. Statistical analysis was performed by a two-sided t-test.

The results demonstrated that cherry blossom extract promotes transcription of these genes, and improves skin barrier function. An OBP expression promoter containing cherry blossom extract can therefore be used to improve skin barrier function. Cherry blossom extract can also be used as an active ingredient in a skin barrier function ameliorating agent.

### Example 6: Amelioration of skin barrier function by lily extract

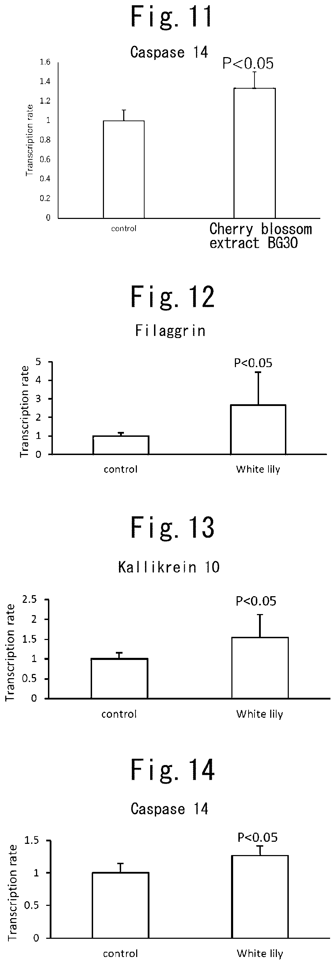

An experiment was conducted in the same manner as Example 5, except that lily extract was used instead of cherry blossom extract. The results are shown in Figs. 12 to 14. The values are represented as relative values with respect to 1 for the control.

The results demonstrated that lily extract promotes transcription of filaggrin, kallikrein 10 and caspase 14, and improves skin barrier function. An OBP expression promoter containing lily extract can therefore be used to improve skin barrier function. Lily extract can also be used as an active ingredient in a skin barrier function ameliorating agent.

### Example 7: Amelioration of transepidermal water loss (TEWL) by cherry blossom extract and lily extract

Skin barrier function can be evaluated by suppression of transdermal water loss, with a lower transepidermal water loss (TEWL) being considered to be superior barrier function.

Human epidermal cells diluted in CnTPrime medium were seeded in a cell culture insert coated with CellStart (Invitrogen Life Technologies) solution diluted 50-fold in DPBS, and CnTPrime medium solution was added under the insert prior to culturing for 3 days at 37°C. The medium on the insert was then replaced with CnT-PR-3D medium containing added vitamin C, and culturing was carried out for 1 day at 37°C. After one day, the medium on the insert was aspirated, and the medium under the insert was replaced with CnT-PR-3D medium containing added vitamin C, and culturing was continued for 1 day at 37°C. This procedure was carried out for 8 days to construct a 3D skin model. Cherry blossom extract or lily extract was added 3 days prior to completion of the skin model, while an equal amount of ethanol was added as solvent for the control, conducting culturing for 3 days at 37°C. The weight of the cultured 3D skin model was periodically measured to determine the TEWL.

The results showed that cherry blossom extract and lily extract significantly lower transepidermal water loss (TEWL). These results also demonstrated that an OBP expression promoter such as cherry blossom extract or lily extract can be used for improvement of skin barrier function.

In the experiments described above, several plant extracts were confirmed to promote expression of OBP. The present invention therefore provides OBP expression promoters containing those substances. The OBP expression promoter may be used for promoting expression of OBP in skin with reduced OBP activity (epidermal cells and/or keratinocytes), or for reducing damage to skin by harmful substances to enhance the moisturizing function and/or barrier function of the skin. The screening method of the invention allows identification of substances that are useful as OBP expression promoters.

## Claims

1. An OBP (odorant binding protein) expression promoter comprising a substance selected from the group consisting of lily extract, cabbage rose extract and cherry blossom extract as an active ingredient.

2. The OBP expression promoter according to claim 1, which is to be used for promoting expression of OBP in epidermal cells and/or keratinocytes.

3. The OBP expression promoter according to claim 1 or 2, which is to be used for promoting expression of OBP in skin with reduced OBP activity.

4. The OBP expression promoter according to any one of claims 1 to 3, which is to be used for enhancing the moisturizing function and/or barrier function of skin.

5. The OBP expression promoter according to any one of claims 1 to 4, which is to be used for reducing damage to skin by harmful substances.

6. The OBP expression promoter according to claim 5, wherein the harmful substance is selected from the group consisting of aldehydes, fatty acids and air pollutants.

7. A cosmetic method or non-therapeutic method that includes application of an OBP expression promoter comprising a substance selected from the group consisting of lily extract, cabbage rose extract and cherry blossom extract as an active ingredient.

8. Use of a substance selected from the group consisting of lily extract, cabbage rose extract and cherry blossom extract for promoting expression of OBP.

9. An external preparation for skin comprising an OBP expression promoter comprising a substance selected from the group consisting of lily extract, cabbage rose extract and cherry blossom extract as an active ingredient.

10. An inhibitor of harmful substances to skin, comprising a substance selected from the group consisting of lily extract, cabbage rose extract and cherry blossom extract as an active ingredient.

11. A method for screening substances that promote or suppress expression of OBP, the method comprises:
a step of contacting cells with a substance,
a step of measuring the expression level of OBP in the cells, and
a step of determining the change in OBP expression level due to the presence or absence of the substance.
